# EUROPEAN PATENT SPECIFICATION

(11) **EP 2 258 416 B1**
(45) Date of publication and mention of the grant of the patent: **26.11.2014**
(21) Application number: 09724051.9
(22) Date of filing: 26.03.2009
(51) Int. Cl.: A61L 33/00, A61F 17/00

(54) **ANTITHROMBOTIC SURFACE**
ANTITHROMBOTISCHE OBERFLÄCHE
SURFACE ANTITHROMBOTIQUE

(30) Priority: 26.03.2008 JP 2008082181
(43) Date of publication of application: 08.12.2010
(73) Proprietor: Kaneka Corporation, Osaka (JP); Snt Co., Chiba 272-0032 (JP)
(72) Inventor: SHIRATORI, Seimei, Ichikawa-shi Chiba 272-0032 (JP); KUWAKI, Oriha, Ichikawa-shi Chiba 272-0032 (JP); KIM, Jin-Ho, Ichikawa-shi Chiba 272-0032 (JP); FUKAYA, Kohei, Settsu-shi Osaka 566-0072 (JP); MIHAYASHI, Tsuyoshi, Settsu-shi Osaka 566-0072 (JP)
(74) Representative: Gille Hrabal
(86) International application number: PCT/JP2009/056164
(87) International publication number: WO 2009/119761

(56) References cited:
- WO-A1-00/13806
- JP-A- 1 299 564
- JP-A- H06 296 682
- JP-T- 2005 538 418
- JP-T- 2007 537 828
- US-A1- 2003 157 260
- S. S. SHIRATORI ET AL: "pH-Dependent Thickness Behavior of Sequentially Adsorbed Layers of Weak Polyelectrolytes", MACROMOLECULES, vol. 33, no. 11, 1 May 2000 (2000-05-01), pages 4213-4219, XP055076724, ISSN: 0024-9297, DOI: 10.1021/ma991645q
- VAZQUEZ-TORRES H ET AL: "POLY(VINYL ALCOHOL)/POLY(ACRYLIC ACID) BLENDS: MISCIBILITY STUDIES BY DSC AND CHARACTERIZATION OF THEIR THERMALLY INDUCED HYDROGELS", JOURNAL OF APPLIED POLYMER SCIENCE, JOHN WILEY & SONS, INC, US, vol. 50, no. 5, 5 November 1993 (1993-11-05), pages 777-792, XP000464170, ISSN: 0021-8995, DOI: 10.1002/APP.1993.070500505
- TOSHIHIRO HIRAI ET AL: "pH-Induced structure change of poly(vinyl alcohol) hydrogel crosslinked with poly(acrylic acid)", DIE ANGEWANDTE MAKROMOLEKULARE CHEMIE, vol. 240, 1 January 1996 (1996-01-01), pages 213-219, XP55076826, ISSN: 0003-3146

## Description

### TECHNICAL FIELD

The present invention relates to a laminated film having a surface superior in biocompatibility, particularly in antithrombogenicity (blood clotting resistance) and additionally to a medical device having a region where the surface thereof is coated with the laminated film.

### BACKGROUND ART

A great number of medical devices have been development recently along with progress in medicine, and medical devices which are brought into contact with blood, such as catheter, artificial heart-lung machine, artificial kidney, artificial cardiac valve, and hemocathartic system, among them, are made of polymer materials and metal materials. These medical devices, when brought into contact with blood, may cause thrombosis by coagulation of the blood on the surface, leading to deterioration in performance during use of the medical devices and further causing clinical problems.

Various antithrombogenic surfaces have been developed to solve the problems above. Examples thereof include methods of immobilizing an antithrombogenic material heparin on surface by various methods, a method of positively charging the surface and making the surface bind with negatively charged heparin, a method of immobilizing heparin, via a linker molecule, to the surface (see, for example, JP 2000279511). However, use of a physiologically active substance, such as heparin, is disadvantageous in many points including that control of production and operation are complicated, applicable surfaces are limited, and the physiologically active substance loses its action, as they are inactivated.

Other known methods include methods of graft-polymerizing a resin such as polyurethane on the surface of a microphase-separated surface or a hydrophilic surface, especially a hydrophilic polymer material (see, for example, JP 60092764). However, the surface of the microphase-separated structure should be controlled in a favorably phase-separated state for expression of favorable antithrombogenicity, and the condition for causing such phase separation is limited. Especially in the case of a urethane resin, it is difficult to make it thin, because it has high viscosity, and it is thus not suited for preparation of fine surfaces. A method of making a material surface hydrophilic and thus improved in biocompatibility by graft polymerization of a hydrophilic monomer such as acrylamide or ethylene glycol on the surface by irradiation of radiation ray or by glow discharge treatment is generally known as the method of preparing a hydrophilic surface. However, the method has many disadvantages, for example, that it is not economical because the apparatus is expensive, it is difficult to make it thin, it is difficult to graft-polymerize uniformly on surfaces, such as internal surface of hollow products and surfaces in complicated shape, and it is also difficult to graft-polymerize the hydrophilic polymer itself.
Patent document JP H06 296682 A describes implants with antithrombogenic ceramic coatings obtained by vapour deposition, sputtering or crystallization. The antithrombogenic property is due to a particular surface structure characterised i.a. by its root mean square average roughness (RMS), which structure reduces the contact area to platelets and thereby thrombosis.
S. S. Shiratori et al. (Macromolecules 33(11), 4213-19 (2000)) teaches that a technique of sequentially adsorbing layers of weak polyelectrolytes of opposite charge can be used to build surfaces of defined thickness and roughness in comparably simple manner, by e.g. pH adjustment.
Patent document US2003/157260 A1 discloses that polyelectrolyte multilayers of for instance poly allyl amine hydrochloride)/poly acrylic acid (PAH/PAA) or poly diallyl dimethyl ammonium chloride/poly acrylic acid (PDDA/PAA) are suitable for coating implants. They can be used to control interaction with cells, introducing desired adhesion of cells and suppressing non-specific adhesion.

### DISCLOSURE OF THE INVENTION

### TECHNICAL PROBLEMS TO BE SOLVED

As described above, although various antithrombogenic surfaces have been studied, surfaces containing a physiologically active substance had disadvantages that the applicable surfaces are limited and measures for preventing inactivation should be taken. It is also difficult to obtain a thin antithrombogenic surface by the method of microphase separation and hydrophilization of a polymeric material, and thus, such a method cannot be used for production of medical devices demanding fine structure and high precision. For example in the case of an intravascular filter, which captures embolus-causing substances, such as vascular wall, thrombus and atheroma, scattered in blood, as it is placed in a blood vessel during treatment of internal blood vessel, it is desired to make the filter region anti-thrombogenic for prevention of the filter itself becoming the cause of thrombus formation. However, the filter should also have properties that the openings therein are very fine at a diameter of approximately 100 µm, and the filter is endurable enough to deformation, because it is conveyed to a desired site in the folded state and expanded at the treatment site. For that reason, application of an antithrombogenic surface of common polymeric material, which normally has a thickness of approximately several µm, is very difficult, because it may cause clogging therein or exfoliation thereof. Alternatively in the case of medical devices, such as endoscopes, for insertion into the body for the purpose of diagnosis or treatment, deposition of thrombus on the lens or the lens-protecting filter leads to deterioration in optical function, i.e., difficulty of observation. Application of an antithrombogenic surface is desirable for prevention thereof, but thin coating that does not exert any adverse influence on transparency and other optical functions is desired.

An object of the present invention, which was made to solve the problems in conventional technology, is to provide an antithrombogenic laminated film having a surface superior in antithrombogenicity and a medical device containing the same.

### MEANS TO SOLVE THE PROBLEMS

After intensive studies to solve the problems above, the inventors have found that it is possible to prepare a laminated film having a surface superior in antithrombogenicity, by laminating films of polymer compounds different in electrification state alternately and forming fine spatial asperities on the surface and made the present invention.

Thus, the invention relates to an antithrombogenic laminated film having alternately laminated polymer layers of polymer compounds different in electrification state, **characterized in that** fine spatial asperities are formed on the surface,
wherein the spatial asperities form a texture structure;
the depth of the spatial asperities is 15 nm to 200 nm;
the peak-to-peak distance of the spatial asperities is 200 nm to 2 µm;
the surface roughness (root mean square average roughness: RMS) of the spatial asperities is 2 nm to 40 nm;
the thickness of the polymer layer is 200 nm is 5000 nm;
the polymer layer has polycation layers and polyanion layers laminated alternately; the polycation layer contains polydiallyldimethylammonium chloride (PDDA); and the polyanion layer contains polyacrylic acid (PAA) and polyvinylalcohol (PVA).

The invention also relates to the antithrombogenic laminated film, wherein the laminated outermost layer contains polyacrylic acid (PAA) and polyvinylalcohol (PVA).

The invention also relates to a medical device, characterized in that a surface of the medical device contains a portion to be coated with the antithrombogenic laminated film.

The invention also relates to the medical device, wherein the medical device is a filter to be placed in blood vessel.

The present invention also relates to the medical device having an optical function to be placed in the body, wherein the region having the optical function contains a portion to be coated with an antithrombogenic laminated film.

The present invention also relates to a method of producing the antithrombogenic laminated film, **characterized in that** laminating films of polymer compounds different in electrification state alternately on the surface of a material to be coated.

### EFFECT OF THE INVENTION

The films of the present inventions (1) to (8), which have alternately laminated polymer layers of polymer compounds different in electrification state and also fine spatial asperities formed on the surface, are laminated films having a surface unprecedentedly higher in antithrombogenicity. Such an antithrombogenic laminated film can be prepared, for example, by alternate adsorption method of depositing polymer compounds different in electrification state alternately, and it is possible to make the film very thin and yet show spatial asperities (texture structure), by adjustment of the concentration, pH value and adsorption period of the polymers used and to provide an antithrombogenic surface having unprecedentedly low thickness and favorable coatability. The texture is regular or irregular "pattern" inherently present on the surface of an object, and in the present invention, a pattern irregular but approximately uniform in size, which is formed by the spatial asperities, is called a three-dimensional texture structure.

The invention provides a medical device having a surface unprecedentedly higher in antithrombogenicity. Because it is possible to form the antithrombogenic laminated film even on the surface of a very thin and fine structure for example by the alternate adsorption method above, it is possible to form an antithrombogenic surface on medical devices that could not be designed, because of difficulty in forming the antithrombogenic surface.

The present invention provides a filter having a surface unprecedentedly higher in antithrombogenicity. Because it is possible to form an antithrombogenic laminated film, even on the surface of a very thin and fine-structure, for example by the alternate adsorption method above, the surface of the filter to be placed in blood vessel for capture of the embolus-causing substances can be coated with a laminated film having an antithrombogenic surface, and it is thus possible to provide a practical antithrombogenic filter resistant to clogging and deformation of filter such as folding and expansion.

The invention provides a medical device having a surface unprecedentedly higher in antithrombogenicity. Because it is possible to form an antithrombogenic laminated film, even on the surface of a very thin and fine structure, for example by the alternate adsorption method above, it is possible to coat the surface of medical devices having an optical function, which is to be placed in the body for diagnosis or treatment, with a laminated film having an antithrombogenic surface and thus to provide a medical device resistant to deterioration in the optical function above.

It is also possible according to the invention to form an unprecedentedly high antithrombogenic surface on a material to be coated easily, and it is possible to form an antithrombogenic surface on the surface of a very thin and fine structure particularly by the alternate adsorption method, because the concentration of the coating solution of a polymeric compound carrying particular charge can be made very low.

### BRIEF DESCRIPTION OF THE DRAWINGS

Figure 1 is a schematic view showing the principle in producing an alternate adsorption film.
Figure 2 is a surface SEM image of Example 1.
Figure 3 is a surface SEM image of Comparative Example 1.
Figure 4 is a surface SEM image of Comparative Example 2.
Figure 5 is a surface SEM image of Comparative Example 3.
Figure 6 is a surface SEM image of Comparative Example 6.
Figure 7 is a surface SEM image of Example 4.
Figure 8 is a surface SEM image of Example 5.
Figure 9 is a surface SEM image of Example 6.
Figure 10 is a surface SEM image of Example 7.
Figure 11 is a surface SEM image of Comparative Example 7.

### BEST MODE OF CARRYING OUT THE INVENTION

Hereinafter, the most favorable embodiments of the antithrombogenic laminated film and the medical device according to the present invention will be described. The present invention relates to an antithrombogenic laminated film having alternately laminated polymer layers of polymer compounds different in electrification state that has fine spatial asperities formed on the surface. The surface can be prepared by the alternate adsorption method of adsorbing polymers different in electrification state alternately, which was developed by G. Decher et al. in 1992 originally as a method of forming a composite organic thin film (Decher G, Hong. J.D. and J. Schmit: Thin Solid Films, 210/211, p.831 (1992)), and a technique of alternating adsorption (layer-by-layer electrostatic self-assembly) is used in the preparative process.

According to the basic method developed by G. Decher et al., an aqueous solution of a positively charged (cationic) electrolyte polymer compound and an aqueous solution of a negatively charged electrolyte (anionic) polymer compound are first prepared separately. A previously surface-charged substrate (material to be coated) is then immersed into these containers alternately, to give a composite organic thin film (alternate adsorption film) that has a multilayer structure formed on a substrate and is yet very thin. For example when a glass plate is used as the material to be coated, the surface of the glass plate is hydrophilized by introduction of OH groups on the surface and made negatively charged as the initial surface charge. When the negatively surface-charged substrate is immersed in the aqueous solution of a positively charged electrolyte polymer compound, the positively charged electrolyte polymer compound is adsorbed on the substrate surface by Coulomb force at least until the surface charge is neutralized, forming a thin film having an extremely thin single-layer thickness in the nanometer order (hereinafter, referred to as ultrathin film). The surface region of the ultrathin film thus formed is positively charged. When the substrate is then immersed into the aqueous solution of a negatively charged electrolyte polymer compound, the negatively charged electrolyte polymer compound is adsorbed by Coulomb force, forming another layer of ultrathin film on the surface of the thin film. In this way, it is possible, by immersing the substrate into two containers alternately, to form ultrathin film layers of the positively charged electrolyte polymer compound and ultrathin film layers of the negatively charged electrolyte polymer compound alternately and thus to form a very thin composite organic thin film having a multilayer structure.

Recently, M.F. Rubner et al. reported a technology of automating production of the alternate adsorption film (A.C. Fou, O. Onitsuka, M. Ferreira, B.R. Hsieh and M.F. Rubner: J. Appl. Phys. 79 (10) 15 May 1996) and proposed the configuration of an automated apparatus for production of the alternate adsorption film. In the apparatus, if used, the substrate used as the material to be coated is immersed into two water baths alternately by means of a robot arm, and alternately adsorbed films are formed automatically on the substrate. In addition, the inventors disclosed a film-forming method of forming a film having a film thickness accurately controlled in WO 00/13806.

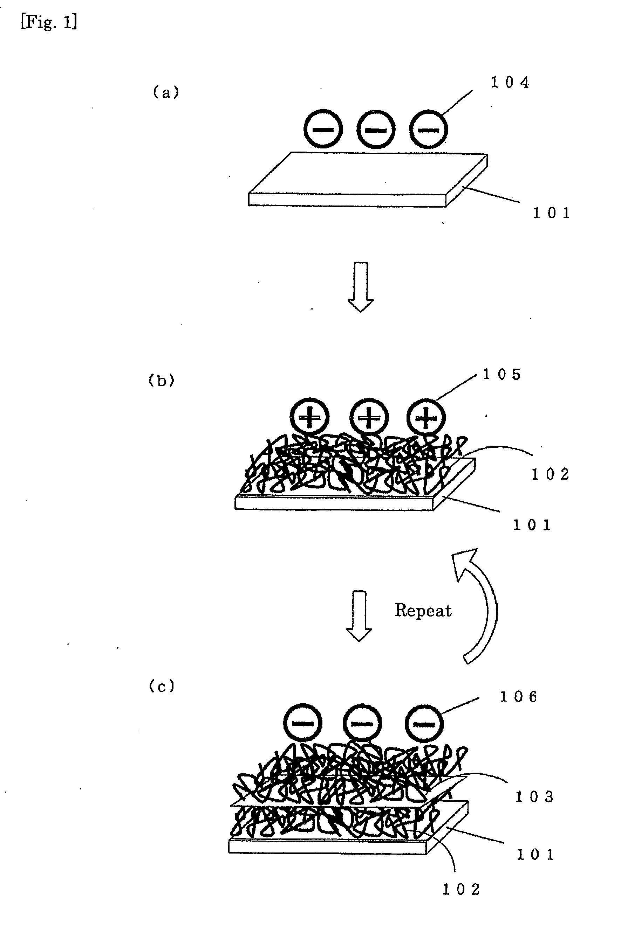

Figure 1 is a schematic view showing the principle in producing the alternate adsorption film. In Figure 1, a substrate 101, for example of glass, silicon or a metal (stainless steel, etc.), is made available as the material to be coated, and the surface thereof is hydrophilized by introduction of OH groups onto the surface and made negatively (-) charged 104 as the initial surface charge (see Figure 1(a)). When the surface of the negatively charged substrate 101 is then brought into contact with a positively charged electrolyte polymer compound, the positively charged electrolyte polymer compound is adsorbed on the surface of the substrate 101 by Coulomb force, forming a polycation layer. Figure 1(b) is a schematic view illustrating a substrate having a positively charged electrolyte polymer compound 102 adsorbed thereon, the outermost layer surface of which is electrified with positive (+) charges 105. When the substrate is then brought into contact with a negatively charged electrolyte polymer compound 103, the negatively charged electrolyte polymer compound is adsorbed by Coulomb force on the surface of the substrate having the formed polycation layer, forming a polyanion layer. Figure 1(c) is a schematic view illustrating the substrate having the negatively charged electrolyte polymer compound 103 adsorbed on the positively charged electrolyte polymer compound 102 as laminated, the outermost layer surface of which is charged with negative (-) charges 106.

When the substrate 101 are brought into contact alternately with polymer compounds carrying different charges in this way, layers of the positively charged electrolyte polymer compound (polycation layer) and layers of the negatively charged electrolyte polymer compound (polyanion layer) are formed alternately on the surface of the substrate 101, finally forming an alternately-adsorbed lamination film having a multilayer structure. Because the repulsion between the segments in the polymer by Coulomb force varies according to conditions such as the concentration, the pH value and the adsorption period of the electrolyte polymer compound used in the adsorption treatment, the packing density of the molecule and its distribution can be controlled by adjustment of these conditions. Thus, it becomes possible to form a very thin film or a relative thick film arbitrarily and to form surface asperities and control the fine spatial asperities, for example texture structure, by properly adjusting the settings of these conditions.

The texture structure is the aggregate of its constituent elements, and the size of the constituent element is not particularly limited, if antithrombogenicity is expressed; but, for improvement of the antithrombogenic effect, the length and the width of the constituent element are both preferably 10 µm or less; more preferably, the length of the constituent element (size of the constituent element in the longer direction) is 3 µm or less and the width (size of the constituent element in the shorter direction) 500 nm or less; and still most preferably, the length and the width of the constituent element are preferably in the range of 3 µm to 50 nm. Although the depth of the spatial asperities may depend on the thickness of the antithrombogenic laminated film, it is preferably 15 nm to 200 nm for effective expression of the antithrombogenic property. Alternatively, the thickness of the alternately absorbed polymer layer, i.e., the antithrombogenic laminated film, is preferably 200 nm to 5000 nm, because the film shows the surface property more easily and is practically more useful.

The dimension of the fine spatial asperities or the texture structure formed on the surface of the antithrombogenic laminated film can be determined by using a laser surface analyzer or a stylus surface profiler (such as DEKTAK, trademark), but it can also be determined, simply by direct observation of the surface and cross section under SEM.

For favorable expression of antithrombogenicity, the peak-to-peak distance of spatial asperities is preferably 200 nm to 2 µm, and a peak-to-peak distance of spatial asperities of less than 200 nm or more than 2 µm may lead to deterioration or disappearance of antithrombogenicity. The peak-to-peak distance of the spatial asperities, which is the distance between neighboring asperity peaks in irregularity, as measured under interatomic microscope, is determined under the "average" measuring condition by using the apparatus and the program described in Examples. For favorable expression of antithrombogenicity, the surface roughness (root mean square average roughness: RMS) of the spatial asperities is preferably 2 nm to 40 nm, and an average roughness of less than 2 nm or more than 40 nm may lead to deterioration or disappearance of antithrombogenicity. The surface roughness of such spatial asperities (root mean square average roughness: RMS) can also be determined similarly under atomic force microscope.

The lamination number is preferably 30 or less, more preferably 20 or less, from the viewpoints of easiness in forming the surface having the properties above and stability thereof. Various polymer compounds different in electrostatic properties can be used as the polymer compounds for lamination, but, when application to medical devices is taken into consideration, use of a polymer compound higher in safety is preferable, and polydiallyldimethylammonium chloride (PDDA) is used favorably as the polycation layer and a mixture of polyacrylic acid (PAA) and polyvinylalcohol (PVA) as the polyanion layer. Alternatively for repulsion of the fibrinogen carrying negative charges, a major causative factor of thrombosis, the outermost layer preferably carries negative charges and is formed in combination of PAA and PVA. The surface is preferably higher in hydrophilicity and thus, the water contact angle of the surface is preferably 60° or lower.

The antithrombogenic laminated film according to the present invention is used for coating the surface of a medical device that may be brought into contact with blood and, in particular, it is used particularly favorably in medical devices that demand a thin and transparent film that is coatable even on fine structures. In particular, a filter placed in blood vessel for capture of embolus-causing substances, which often cause thrombosis, because the filter is placed, as expanded, in blood flow where blood cells flow through fine pore regions at high speed with the blood cells colliding to the pores, should satisfy the requirements that the diameter of the pore region is very fine at a diameter of approximately 100 µm and the filter is resistant enough to the deformation during conveyance in the folded state to a desired site and expansion at the treatment site. Thus, it is very difficult to use an antithrombogenic surface made of a film of common polymeric material, which has a thickness of several µm, because it may cause clogging on the surface or exfoliation thereof. For that reason, the antithrombogenic laminated film according to the present invention is used favorably.

Further in the case of medical devices for insertion into the body for diagnosis or treatment such as endoscope, deposition of thrombus on the lens or the lens-protecting filter leads to deterioration in optical function, i.e., difficulty of observation. It is needless to say that the surface may be coated to form an antithrombogenic surface for prevention thereof but the surface should be sufficiently transparent for prevention of deterioration in observation efficiency. Medical devices for insertion into the body are often small-diameter devices and the lenses therein, for example, are also very small, and thus, they are easily influenced by the thickness of the coat, if it is formed. Thus, they demand transparent and thin coating, and the antithrombogenic laminated film according to the present invention is used favorably.

### EXAMPLES

Hereinafter, more specific Examples of the present invention will be described more in detail.

### (Example 1)

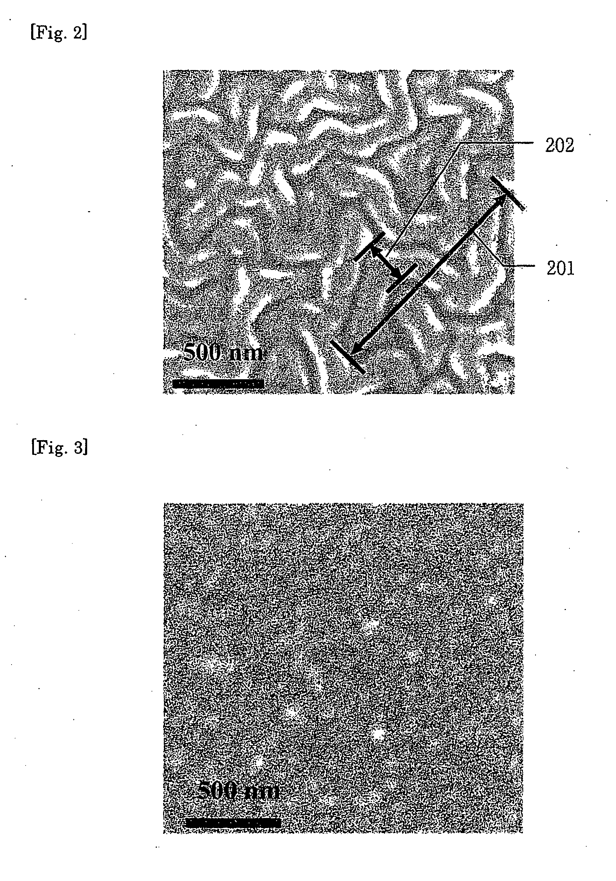

A glass plate previously charged negatively was immersed in 100 mM polydiallyldimethylammonium chloride (PDDA) solution for 15 minutes, forming a polycation layer; it was then immersed in purified water for cleaning thrice for 2 minutes, 1 minute and 1 minute; it was then immersed in 1:1 mixture solution of polyvinylalcohol (PVA: molecular weight: 1500, 1 wt %) and polyacrylic acid (PAA: molecular weight: 90000, 20 mM), forming a polyanion layer; and it was then immersed in purified water for cleaning thrice for 2 minutes, 1 minute and 1 minute. The operation above was repeated 20 times (lamination number: 20), forming a PVA/PAA surface having a negatively charged outermost layer on the glass plate. The SEM image of the surface is shown in Figure 2. As shown in Figure 2, the surface had a three-dimensional texture structure having surface irregularity. Subsequently, the length 201 of the constituent elements in the three-dimensional texture structure, as determined under SEM, was averagely 3 µm or less; the width 202 was averagely 500 nm or less; the surface irregularity in the depth direction was 200 nm; and the thickness of the alternately laminated polymer layer was 5000 nm.

### (Comparative Example 1)

A glass plate previously charged negatively was immersed in 100 mM polydiallyldimethylammonium chloride (PDDA) solution for 15 minutes, forming a polycation layer; it was then immersed in purified water for cleaning thrice for 2 minutes, 1 minute and 1 minute; it was then immersed in a solution of polystyrene sulfone (PSS: molecular weight: 70000, concentration: 100 mM) for 15 minutes, forming a polyanion layer; and it was then immersed in purified water for cleaning thrice for 2 minutes, 1 minute and 1 minute. The operation above was repeated 20 times (lamination number: 20), forming a PSS surface having a negatively charged outermost layer on the glass plate. The SEM image of the surface is shown in Figure 3. As shown in the figure, the surface was not irregular and smooth.

### (Comparative Example 2)

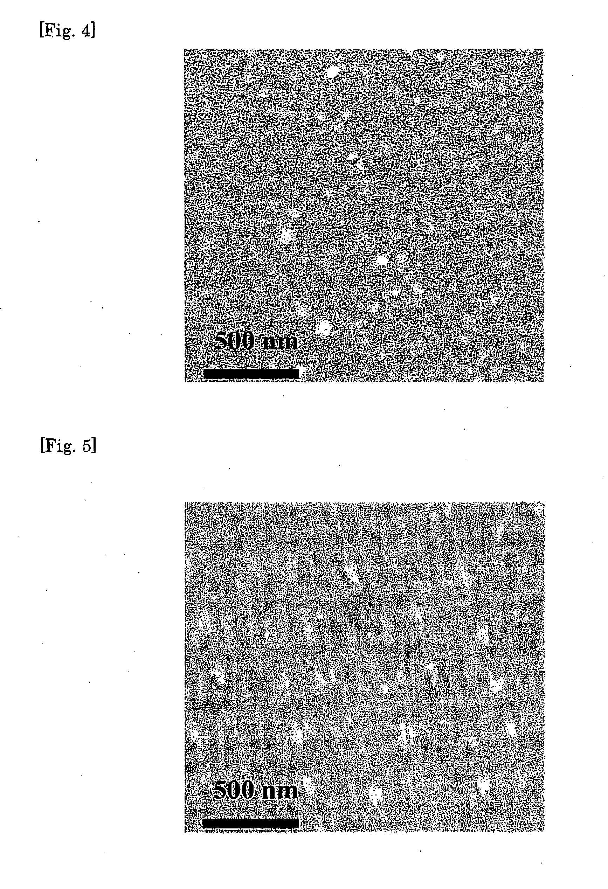

A glass plate previously charged negatively was immersed in 100 mM polydiallyldimethylammonium chloride (PDDA) solution for 15 minutes, forming a polycation layer; it was then immersed in purified water for cleaning thrice for 2 minutes, 1 minute and 1 minute; it was then immersed in a solution of titanium (IV) bis(ammonium lactato)dihydroxide (TALH: concentration: 1 wt %) for 15 minutes, forming a polyanion layer; and it was then immersed in purified water for cleaning thrice for 2 minutes, 1 minute and 1 minute. The operation above was repeated 19 times, and then, treated with the solution of polydiallyldimethylammonium chloride (PDDA), to give a PDDA surface having a positively charged outermost layer. The SEM image of the surface is shown in Figure 4. As shown in the figure, the surface was not irregular and smooth.

### (Comparative Example 3)

A glass plate previously charged negatively was immersed in 100 mM polydiallyldimethylammonium chloride (PDDA) solution for 15 minutes, forming a polycation layer; it was then immersed in a mixed solution of a 1:1 mixture solution of polyvinylalcohol (PVA: molecular weight: 1500, 1 wt %) and polyacrylic acid (PAA: molecular weight: 90000, 20 mM) with gultaraldehyde (finally 3 wt % in the mixed solution) for 15 minutes, forming a polyanion layer; and it was then immersed in purified water for cleaning thrice for 2 minutes, 1 minute and 1 minute. The operation above was repeated 20 times, to give a PVA/PAA surface having a negatively charged outermost layer on the glass plate. The SEM image of the surface is shown in Figure 5. As shown in the figure, the surface was not irregular and smooth.

### (Comparative Example 4)

The glass plate used in Example 1 (without treatment) was used for comparison.

### (Example 2)

A PVA/PAA surface having a three-dimensional texture structure having a negatively charged outermost layer and an irregular surface similar to that in Example 1 was formed on the filter region of a catheter-shaped medical device previously negatively charged that is to be installed in blood vessel for capturing embolus-causing substances (304 stainless steel, diameter 20 µm, opening: 100 µm) at a lamination number of 20, by an operation similar to that in Example 1. The length of the constituent elements in the three-dimensional texture structure, as determined then under SEM, was averagely 3 µm or less; the width was averagely 500 nm or less; the surface irregularity in the depth direction was 70 nm; and the thickness of the alternately laminated polymer layer was 1000 nm.

### (Example 3)

A PVA/PAA surface having a three-dimensional texture structure having a negatively charged outermost layer and an irregular surface, similar to that in Example 1 was formed on the filter region of a catheter-shaped medical device previously negatively charged that is to be installed in blood vessel for capturing embolus-causing substances (304 stainless steel, diameter 20 µm, opening: 100 µm) at a lamination number of 5 by an operation similar to that in Example 1. The length of the constituent elements in the three-dimensional texture structure, as determined then under SEM, was averagely 3 µm or less; the width was averagely 500 nm or less; the surface irregularity in the depth direction was 15 nm; and the thickness of the alternately laminated polymer layer was 200 nm.

### (Comparative Example 5)

A PVA/PAA surface having a negatively charged outermost layer but having no irregularity, which is similar to that in Example 3, was prepared on the filter region of a catheter-shaped medical device previously negatively charged that is to be installed in blood vessel for capturing embolus-causing substances (304 stainless steel, diameter 20 µm, opening: 100 µm), by an operation similar to that in Comparative Example 3, except that the immersion number was 5.

### (Comparative Example 6)

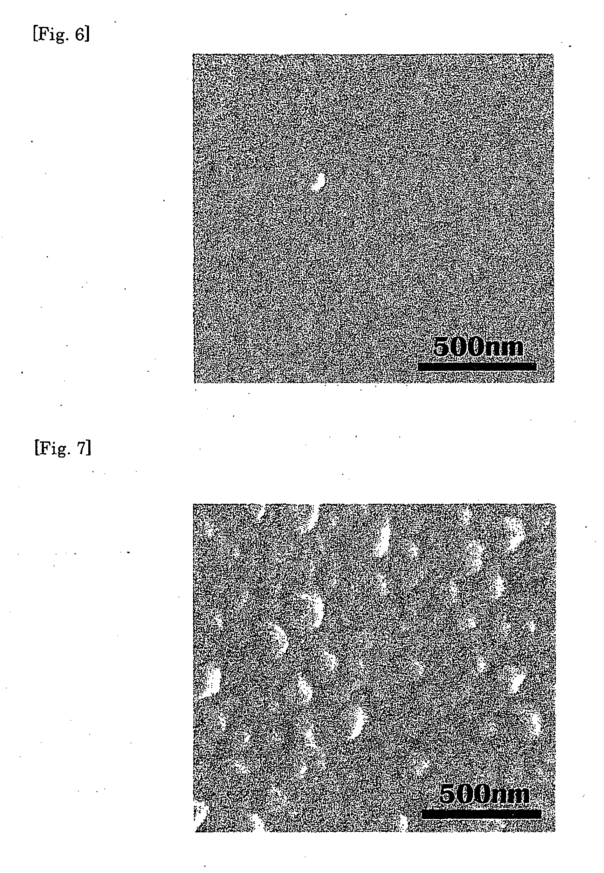

The filter used in Example 2 (without treatment) was used for comparison. The SEM image of the surface is shown in Figure 6.

### (Example 4)

Operation similar to that in Example 2, except that the lamination number was 3, gave a filter covered with an antithrombogenic laminated film having a PVA/PAA surface in a three-dimensional texture structure having a negatively charged outermost layer and an irregular surface, similar to that in Example 1, on the filter. The length of the constituent elements in the three-dimensional texture structure, as determined then under SEM, was averagely 3 µm or less and the width, averagely 500 nm or less. The SEM image of the surface is shown in Figure 7. The peak-to-peak distance of spatial asperities (average) of the three-dimensional texture structure, as determined under atomic force microscope (Atomic Force Microscope NanoScope IIIa, manufactured by Digital Instruments, analytical software NanoScope IIIa, Version 5.30 r3sr3, manufactured by Veeco Instruments) was 200 nm, the surface irregularity in the depth direction (bearing) was 15.8 nm; and the surface roughness (root mean square average roughness: RMS) was 2.47 nm.

### (Example 5)

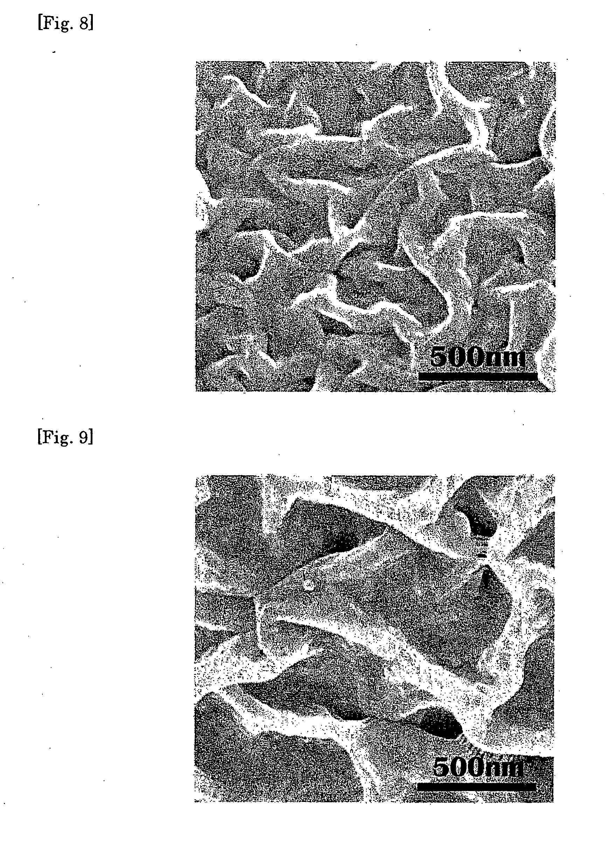

Operation similar to that in Example 2, except that the lamination number was 10, gave a filter covered with an antithrombogenic laminated film having a PVA/PAA surface in a three-dimensional texture structure having a negatively charged outermost layer and an irregular surface, similar to that in Example 1 on the filter. The length of the constituent elements in the three-dimensional texture structure, as determined then under SEM, was averagely 3 µm or less and the width, averagely 500 nm or less. The SEM image of the surface is shown in Figure 8. The peak-to-peak distance of spatial asperities (average) of the three-dimensional texture structure, as determined under atomic force microscope in a manner similar to Example 4, was 1 µm; the surface irregularity in the depth direction (bearing) was 133 nm; and the surface roughness (root mean square average roughness: RMS) was 37.6 nm.

### (Example 6)

Operation similar to that in Example 2, except that the lamination number was 15, gave a filter covered with an antithrombogenic laminated film having a PVA/PAA surface in a three-dimensional texture structure having a negatively charged outermost layer and an irregular surface, similar to that in Example 1, on the filter. The length of the constituent elements in the three-dimensional texture structure, as determined then under SEM, was averagely 3 µm or less and the width, averagely 500 nm or less. The SEM image of the surface is shown in Figure 9. The peak-to-peak distance of spatial asperities (average) of the three-dimensional texture structure, as determined under atomic force microscope in a manner similar to Example 4, was 2 µm; the surface irregularity in the depth direction (bearing) was 42.2 nm; and the surface roughness (root mean square average roughness: RMS) was 33.0 nm.

### (Example 7)

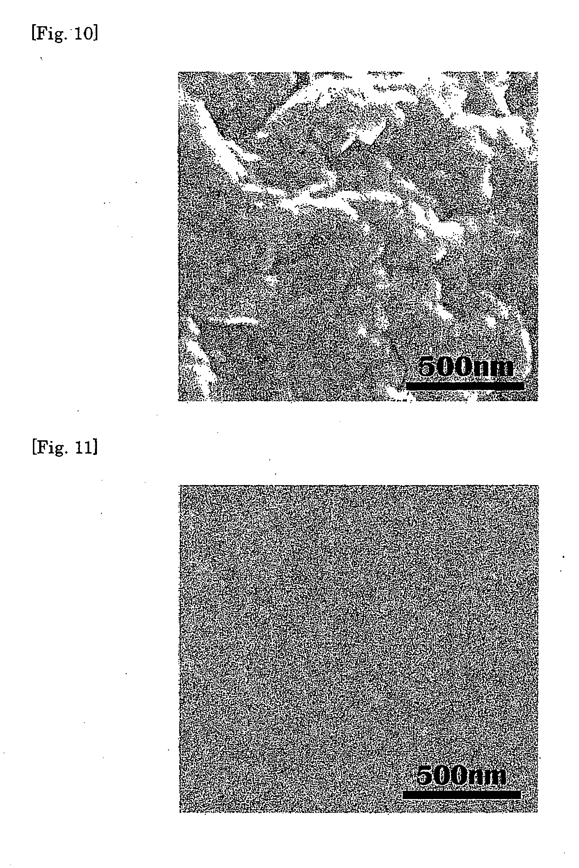

Operation similar to that in Example 2, except that the lamination number was 30, gave a filter covered with an antithrombogenic laminated film having a PVA/PAA surface in a three-dimensional texture structure having a negatively charged outermost layer and an irregular surface, similar to that in Example 1, on the filter. The length of the constituent elements in the three-dimensional texture structure, as determined then under SEM, was averagely 4 µm or more and the width, averagely 500 nm or more. The SEM image of the surface is shown in Figure 10. The peak-to-peak distance of spatial asperities (average) of the three-dimensional texture structure, as determined under atomic force microscope in a manner similar to Example 4, was 7.5 µm. The surface irregularity in the depth direction (bearing) was 14 nm, and the surface roughness (root mean square average roughness: RMS) was 35.5 nm.

### (Comparative Example 7)

Operation similar to that in Example 2, except that the lamination number was 1, gave a filter covered with an antithrombogenic laminated film having a PVA/PAA surface in a three-dimensional texture structure having a negatively charged outermost layer and an irregular surface, similar to that in Example 1, on the filter. The three-dimensional texture structure was not observed then under SEM. The SEM image of the surface is shown in Figure 11. The peak-to-peak distance of spatial asperities (average) of the three-dimensional texture structure, as determined under atomic force microscope in a manner similar to Example 4, was unmeasurable; the surface irregularity in the depth direction (bearing) was 7.8 nm; and the surface roughness (root mean square average roughness: RMS) was 1.46 nm.

### (Evaluation 1)

Each of the antithrombogenic surfaces obtained in Example 1 and Comparative Examples 1 to 4 was brought into contact with human blood plasma-derived fibrinogen for 180 minutes, and the resulting deposition of fibrinogen was evaluated by visual observation and also by measurement of surface zeta potential and light transmittance. There was no deposition of fibrinogen on the surface of Example 1 by visual observation; the surface zeta potential thereof showed no change from -30 mV before contact; and the light transmittance also remained without change, indicating that there was no deposition of fibrinogen in the first phase of thrombus formation. There was deposition of fibrinogen on the surface of Comparative Example 1 by visual observation; the surface zeta potential changed to -5 mV, zeta potential characteristic of fibrinogen, from -70 mV before contact; and the light transmittance also dropped by approximately 5%. There was deposition of fibrinogen on the surface of Comparative Example 2 by visual observation; the surface zeta potential changed to -5 mV, zeta potential characteristic of fibrinogen, from +40 mV before contact; and the light transmittance also dropped by approximately 5%. There was deposition of fibrinogen on the surface of Comparative Example 3 by visual observation; the surface zeta potential changed to -5 mV, zeta potential characteristic of fibrinogen, from -25 mV before contact; and the light transmittance also dropped by approximately 5%. There was deposition of fibrinogen on the surface of Comparative Example 4 by visual observation; the surface zeta potential changed to -5 mV, zeta potential characteristic of fibrinogen, from -80 mV before contact; and the light transmittance also dropped by approximately 5%. These results indicate that the antithrombogenic surface of Example 1 shows antithrombogenicity, the advantageous effect of the present invention.

### (Evaluation 2)

A blood-flow circuit of a silicone tube having an inner diameter of 3 mm for circulation of infant swine blood from carotid artery to vein was constructed. Filters for evaluation, specifically the filters of Examples 2 and 3 and Comparative Examples 5 and 6, were installed one by one in the silicone tube, while ACT was controlled to 100 to 200 by heparin administration. After blood circulation, the blood flow rate was monitored for the estimated operating period of catheter-type filter of 15 minutes, by using an electromagnetic flowmeter. The period until blood flow is terminated by the filter clogging caused by thrombus formation and the state of thrombus formation on the filter at the time then were examined. In the case of the filters obtained in Examples 2 and 3, there was no change in blood flow rate from approximately 130 ml/minute immediately after initiation of blood circulation for a period of 15 minutes, and there was no thrombus formation on the filter, even after the evaluation test. In the case of the filter of Comparative Example 5, the blood flow rate decreased to 0 within 2 minutes from approximately 130 ml/minute immediately after initiation of blood circulation and the filter after the evaluation test was clogged with the thrombus formed. In the case of the filter of Comparative Example 6, the blood flow rate decreased to approximately 70 ml/minute within 2 minutes and to 0 within 5 minutes from approximately 130 ml/minute immediately after initiation of blood circulation. The filter after the evaluation test was clogged with the thrombus formed.

These results demonstrated that the filters, i.e., the medical devices to which the antithrombogenic surfaces of Examples 2 and 3 in the present invention are applied, can function as a filter superior in antithrombogenicity, showing the advantageous effects of the present invention.

### (Evaluation 3)

A blood-flow circuit of a silicone tube having an inner diameter of 3 mm for circulation of infant swine blood from carotid artery to vein was constructed. The filters for evaluation, specifically the filters of Examples 4, 5, 6 and 7 and Comparative Examples 6 and 7, were installed one by one in the silicone tube, while ACT was controlled to 100 to 150 by heparin administration. After blood circulation, the blood flow rate was monitored for the estimated operating period of the catheter-type filter of 15 minutes by using an electromagnetic flowmeter. The period until the blood flow rate was reduced to half by the filter clogging caused by thrombus formation was determined. In the case of the filters of Examples 4, 5 and 6, there was no incidence of reduction of blood flow rate to half within 15 minutes after initiation of blood circulation and no thrombus was formed on the filter even after the evaluation test. In the case of the filter of Example 7, the blood flow rate declined to half within 14 minutes after initiation of blood circulation, but the filter was not clogged for the estimated operation period of the filter of 15 minutes. Alternatively in the case of the filter of Comparative Example 6, the blood flow rate declined to half within 2 minutes after initiation of blood circulation. In the case of the filter of Comparative Example 7, the blood flow rate declined to half within 6 minutes after initiation of blood circulation. Alternatively in the case of any one of the filters of Comparative Examples, the filter was clogged completely and became unusable within the filter's estimated operating period of 15 minutes.

These results demonstrated that the filters, medical devices to which the laminated films of Examples 4, 5 and 6 in the present invention is applied, can function as a filter superior in antithrombogenicity and show the advantageous effects of the present invention.

### EXPLANATION OF REFERENCES

101: Substrate
102: Positively charged electrolyte polymer compound
103: Negatively charged electrolyte polymer compound
104: Negative (-) charge
105: Positive (+) charge
106: Negative (-) charge
201: Length of the constituent element of three-dimensional texture structure
202: Width of the constituent element of three-dimensional texture structure

## Claims

1. An antithrombogenic laminated film having alternately laminated polymer layers of polymer compounds different in electrification state, **characterized in that** fine spatial asperities are formed on the surface, wherein
the spatial asperities form a texture structure;
the depth of the spatial asperities is 15 nm to 200 nm;
the peak-to-peak distance of the spatial asperities is 200 nm to 2 µm; the surface roughness (root mean square average roughness: RMS) of the spatial asperities is 2 nm to 40 nm;
the thickness of the polymer layer is 200 nm is 5000 nm; and
the polymer layer has polycation layers and polyanion layers laminated alternately; the polycation layer contains polydiallyldimethylammonium chloride (PDDA); and the polyanion layer contains polyacrylic acid (PAA) and polyvinylalcohol (PVA).

2. The antithrombogenic laminated film according to Claim 1, wherein the laminated outermost layer contains polyacrylic acid (PAA) and polyvinylalcohol (PVA).

3. A medical device, **characterized in that** a surface of the medical device contains a portion to be coated with the antithrombogenic laminated film according to any one of Claims 1 and 2.

4. The medical device according to Claim 3, wherein the medical device is a filter to be placed in blood vessel.

5. The medical device according to Claim 3 having an optical function to be placed in the body, wherein the region having the optical function contains a portion to be coated with an antithrombogenic laminated film.

6. A method of producing the antithrombogenic laminated film according to any one of Claims 1 and 2, **characterized in that** laminating films of polymer compounds different in electrification state alternately on the surface of a material to be coated.

## Patentansprüche

1. Laminierter antithrombogener Film mit abwechselnd laminierten Polymerschichten von Polymerverbindungen, welche sich im Elektrifizierungszustand unterscheiden, **dadurch gekennzeichnet, dass** auf der Oberfläche feine räumliche Oberflächenunebenheiten gebildet werden, worin
die räumlichen Oberflächenunebenheiten eine Texturstruktur bilden;
die Tiefe der räumlichen Oberflächenunebenheiten 15 nm bis 200 nm beträgt;
die Distanz von Spitze zu Spitze der räumlichen Oberflächenunebenheiten 200 nm bis 2 µm beträgt;
die Oberflächenrauheit (Quadratmittel der Rauheit: RMS) der räumlichen Oberflächenunebenheiten 2 nm bis 40 nm beträgt;
die Dicke der Polymerschicht 200 nm bis 5000 nm beträgt; und
die Polymerschicht Polykationschichten und Polyanionschichten abwechselnd laminiert aufweist; die Polykationschicht Polydiallyldimethylammoniumchlorid (PDDA) enthält; und die Polyanionschicht Polyacrylsäure (PAA) und Polyvinylalkohol (PVA) enthält.

2. Laminierter antithrombogener Film nach Anspruch 1, worin die äußerste laminierte Schicht Polyacrylsäure (PAA) und Polyvinylalkohol (PVA) enthält.

3. Medizinische Vorrichtung, **dadurch gekennzeichnet, dass** eine Oberfläche der medizinischen Vorrichtung einen Teil enthält, welcher mit dem laminierten antithrombogenen Film nach einem der Ansprüche 1 oder 2 beschichtet werden soll.

4. Medizinische Vorrichtung nach Anspruch 3, worin die medizinische Vorrichtung ein Filter ist, welcher in ein Blutgefäß platziert werden soll.

5. Medizinische Vorrichtung nach Anspruch 3 mit einer optischen Funktion, welche in den Körper platziert werden soll, worin die Region mit der optischen Funktion einen Teil enthält, welcher mit einem laminierten antithrombigenen Film beschichtet werden soll.

6. Verfahren zur Herstellung des laminierten antithrombogenen Films nach einem der Ansprüche 1 oder 2, **dadurch gekennzeichnet, dass** Filme von Polymerverbindungen, welche sich im Elektrifizierungszustand unterscheiden, abwechselnd auf die Oberfläche des zu beschichtenden Materials laminiert werden.

## Revendications

1. Film stratifié antithrombogène ayant des couches de polymère stratifiées en alternance des composés de polymère dans un état d'électrification différent, **caractérisé en ce que** des aspérités fines spatiales sont formées sur la surface, dans lequel les aspérités spatiales forme une structure de texture ;
la profondeur des aspérités spatiales est de 15 nm à 200 nm ;
la distance de pic à pic des aspérités spatiales est de 200 nm à 2 µm ;
la rugosité de la surface (valeur quadratique moyenne de la rugosité : RMS) des aspérités spatiales est de 2 nm à 40 nm ;
l'épaisseur de la couche de polymère est de 200 nm à 5000 nm ; et
la couche de polymère présente des couches polycationiques et des couches polyanioniques stratifiées en alternance ; la couche polycationique contient du chlorure de polydiallyldiméthylammonium (PDDA) ; et la couche polyanionique contient de l'acide polyacrylique (PAA) et de l'alcool polyvinylique (PVA).

2. Film stratifié antithrombogène selon la revendication 1, dans lequel la couche stratifiée extrinsèque contient de l'acide polyacrylique (PAA) et de l'alcool polyvinylique (PVA).

3. Dispositif médical, **caractérisé en ce qu'**une surface du dispositif médical contient une portion qui doit être revêtue par le film stratifié antithrombogène selon l'une des revendications 1 ou 2.

4. Dispositif médical selon la revendication 3, dans lequel le dispositif médical est un filtre qui doit être placé dans un vaisseau sanguin.

5. Dispositif médical selon la revendication 3 ayant une fonction optique qui doit être placée dans le corps, dans lequel la région ayant la fonction optique contient une portion qui doit être revêtue par un film stratifié antithrombogène.

6. Procédé de production du film stratifié antithrombogène selon l'une des revendications 1 à 2, **caractérisé en ce que** des couches des composés de polymère dans un état d'électrification différent sont stratifiées en alternance sur la surface d'un matériau qui doit être revêtu.
